(19) **Europäisches Patentamt European Patent Office Office européen des brevets**

(11) **EP 4 408 521 B1**

(12) **EUROPEAN PATENT SPECIFICATION**

(45) Date of publication and mention of the grant of the patent:
**11.03.2026 Bulletin 2026/11**

(21) Application number: **22873731.8**

(22) Date of filing: **27.09.2022**

(51) International Patent Classification (IPC):
*A61N 1/40* (2006.01)    *A61N 2/02* (2006.01)
*A61N 1/36* (2006.01)

(52) Cooperative Patent Classification (CPC):
**A61N 2/02; A61N 1/36002; A61N 1/40**

(86) International application number:
**PCT/US2022/044834**

(87) International publication number:
**WO 2023/049492 (30.03.2023 Gazette 2023/13)**

(54) **INDUCED ELECTRIC FIELD (IEF) THERAPY FOR TREATMENT OF SOLID CANCERS**

THERAPIE MIT INDUZIERTEM ELEKTRISCHEM FELD (IEF) ZUR BEHANDLUNG VON SOLIDEN TUMOREN

THÉRAPIE PAR CHAMP ÉLECTRIQUE INDUIT (IEF) POUR LE TRAITEMENT DE CANCERS SOLIDES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.09.2021 US 202163248694 P**
**14.01.2022 US 202263299668 P**

(43) Date of publication of application:
**07.08.2024 Bulletin 2024/32**

(73) Proprietor: **Ohio State Innovation Foundation Columbus, OH 43210 (US)**

(72) Inventors:
• **AHIRWAR, Dinesh**
**Columbus, Ohio 43201 (US)**
• **JONES, Travis H.**
**Columbus, Ohio 43201 (US)**
• **KAUL, Kirti**
**Columbus, Ohio 43201 (US)**
• **SONG, Jonathan W.**
**Columbus, Ohio 43201 (US)**
• **GANJU, Ramesh**
**Columbus, Ohio 43201 (US)**
• **SUBRAMANIAM, Vishwanath**
**Columbus, Ohio 43201 (US)**
• **CHARAN, Manish**
**Columbus, Ohio 43201 (US)**

(74) Representative: **Berggren Oy**
**P.O. Box 16**
**Fabianinkatu 21**
**00101 Helsinki (FI)**

(56) References cited:
WO-A1-2019/142196    WO-A1-2021/149864
WO-A1-2021/149864    KR-A- 20110 085 708
US-A- 4 998 532      US-A1- 2019 299 019
US-B1- 6 186 941     US-B2- 10 556 121
US-B2- 11 071 875    US-B2- 7 758 490
US-B2- 9 844 347

**Description**

**BACKGROUND**

Breast cancer is a major cause of cancer-related mortalities in women. Among the different subtypes of breast cancer, triple-negative breast cancer (TNBC) correlates with poor prognosis and worse survival due to early metastasis to other organs and lack of clinically established targeted therapies. Triple-negative breast cancer (TNBC) remains the most challenging type of breast cancer compared to other breast cancer subtypes. Tumor-associated macrophages (TAMs) are important tumor-promoting cells in the breast tumor microenvironment (TME). Macrophages account for approximately 30-50% of immune cells in the TME. TAMs are reported to enhance breast tumor progression, including, invasion, and metastasis. In addition, TAMs play a key role in developing therapy resistance in breast cancer models. TAMs also increase tumor angiogenesis and helps in creating an immunosuppressive TME. Macrophages are well known for their plastic nature, which is usually exploited by cancer cells that polarize classic macrophages (M1) to an immunosuppressive phenotype known as M2 TAMs and create a microenvironment to support tumor progression and metastasis.

Increased recruitment of TAMs correlates with a worse prognosis of breast tumors. The intervention strategies against TAMs could be a reduction of macrophage recruitment to tumors or altering their polarization from M2 TAMs to an antitumor M1 phenotype. TAMs regulate a wide array of functions in the TME including tumor invasion, metastasis, and immune responses. M2 TAMs have been shown to inhibit the infiltration and activation of CD8+ T cells in the TME. Tumor-reactive CD8+ T cells predominantly aid in the clearance of tumor cells. Notably, depletion of macrophages has been shown to improve the infiltration of CD8+ T cells, which underscores the impact of macrophages in impeding tumor immunity.

Non-contact induced electric fields (iEFs) generated by time-dependent magnetic fields produced by current-carrying coils driven at 100 kHz have been shown to selectively hinder the migration of highly metastatic breast cancer cells *in vitro.* Similar effects have been observed *in vitro* with other cells such as keratinocytes and murine wound macrophages. These iEFs have been shown in a direction-dependent manner, to either slow down or completely arrest some of the time, migrating cancer cells in microchannels mimicking the topography of preexisting paths formed by vessels, extracellular matrix fibers, and white matter tracts in the brain that guide migrating cancer cells in vivo. Further, this slowing down and arrest of migrating cancer cells has been shown to be associated with changes in metabolic activity. Specifically, iEFs lowered the activity of succinate dehydrogenase (SDH) selectively *in vitro* in highly metastatic MDA-MB-231 breast cancer cells while also selectively increasing the activity of lactate dehydrogenase (LDH) in non-transformed, normal MCF10A breast epithelial cells. SDH is a surrogate marker of oxidative phosphorylation that occurs in the mitochondria of cells and LDH is a surrogate marker of glycolysis that occurs in the cytoplasm of cells. These changes in metabolic activity also serve to explain previously reported changes in the actin cytoskeleton of these breast cancer cells, which is also linked to changes in metabolic activity leading to altered ATP production.

In addition to these previously reported in vitro results, there has been a recent report of the effects of electromagnetic fields on treating type 2 diabetes in mouse models. These demonstrate in diabetic mouse models the use of "static" (i.e., time-invariant or non-time dependent) electric and magnetic fields to modulate redox systems for the non-invasive treatment of type 2 diabetes. However, these do not teach that their vertically directed static electric field and laterally directed static magnetic field are not static at all. While it is true that they are static (steady) in the laboratory frame of reference, they are dynamic (time-varying) in the frame of reference of the mice as the mice move about inside the cage. In other words, with respect to the moving mice, the magnetic field appears to be changing with time and will therefore induce an electric field (effectively an iEF) in the mice whose magnitude and direction will depend on the velocity of the mice. This can be seen more succinctly in Faraday's law:

$$\vec{\nabla} \times \vec{E} = -\frac{D\vec{B}}{Dt} = -\frac{\partial \vec{B}}{\partial t} - (\vec{V}_{mouse} \cdot \vec{\nabla})\vec{B} = -(\vec{V}_{mouse} \cdot \vec{\nabla})\vec{B}$$

where D/Dt denotes the substantial or material derivative (i.e., in a frame of reference moving with the mice), $\vec{E}$ is the induced electric field and $\vec{B}$ is the (spatially inhomogeneous) magnetic induction applied in the laboratory frame and is hence, steady. In addition to the iEF, $\vec{E}$, a static electric field $\vec{E}_{applied}$ may be applied so that experiments have a dynamic electric field $\vec{E}_{applied}(x, y, z) + \vec{E}(x, y, z, t),$ where (x,y,z) are the position coordinates and t is time.

Numerous processes involved in cell migration including actomyosin contraction, focal adhesion point breaks, actin polymerization are known to be energy-intensive processes that rely primarily on mitochondrial respiration. Migration characteristics such as cell speed and net displacement are dependent on ATP:ADP ratio in a cell. While other energetic pathways such as glycolysis and pentose phosphate shunt also contribute to supplying the cell with energy, mitochondrial oxidative phosphorylation is the major contributor. Moreover, mitochondrial function also controls $Ca^{2+}$ channels and reactive oxygen species generation, both of which act as secondary messengers in the pathways involved in cell migration, both in physiologically normal and cancer cells. Although conventionally considered glycolytic, most cancer cells meet their energy needs through mitochondrial function. Furthermore, the significance of the mitochondrial respiratory enzyme succinate dehydrogenase (SDH) (Complex II of the electron transport chain) is best appreciated when considering tumors bearing mutations in SDH. Several studies on MDA-MB-231 cells targeting mitochondrial fusion or fission have found a significant reduction in cell migration, invasion, and proliferation.

It has also recently been shown that oxidative phosphorylation in the mitochondria of metastatic breast cancer cells (MDA-MB-231) is suppressed by iEFs but not in the normal MCF10A breast epithelial cells. This has strong implications for hindering metastasis, which is an energy-intensive process. Though cancer cells are known to rely on other metabolic pathways such as glycolysis, fatty acid metabolism, and glutamine metabolism, they still rely primarily on oxidative phosphorylation to generate the energy required for metastasis. Therefore, iEFs can be effective in attacking the most energetic pathways that cancer cells use to metastasize to distant sites.

WO 2021/149864 discloses an electric field-based cancer treatment apparatus that includes a coil-type conductor to apply an induced electromotive force to a patient's body. The force is induced through a magnetic flux change made by the coil-type conductor when an alternating current is applied to it (par. [0021]. This apparatus is designed to maximize the electric field inside the human body while maintaining a low skin current, enhancing cancer treatment and reducing side effects like skin burns (pars. [0001], [0031]).

US 2019/299019 discloses a method and apparatus for treatment of cancer. The method includes systemic delivery of A3 selective adenosine receptor (A3AR) agonists and providing simultaneous pulsed Electric Field (EF) stimulation to the treatment area. The EF stimulation can be delivered in three orthogonal directions, which significantly increases expression of A3ARs on the cellular membranes and allows achieving a downstream apoptotic signal that is 4-6 -fold stronger than the signal achieved with the agonist drugs alone (see pars. [0039], [0044]).

It is with respect to these and other considerations that the various aspects and embodiments of the present disclosure are presented.

## SUMMARY

The invention is defined by independent claim 1. This invention is directed to systems that use induce electric fields or result in induced electric fields (iEFs) to control and hinder metastatic disease, such as invasive solid cancers.

*In vitro* and *in vivo* results to date indicate that iEF treatment can be used to control and hinder metastatic disease without any accompanying adverse effects. The translation of iEF treatment to higher animals and humans can potentially be enabled in several forms.

An implementation for treatment involves an external, portable induction coil with its associated power source. The patient continuously (or for a predetermined duration) wears the external power supply and iEF coil until treatment is completed as determined by standard diagnostic techniques (CT scans, PET scans, ultrasound, biopsy, etc.).

Another implementation is the coil generating the iEF (and its associated power supply) being an integral part of a fabric worn by the patient.

Yet another implementation involves iEF generating coils embedded in a mattress (or other pieces of furniture or objects), on which a patient would sleep or rest.

Yet another implementation is where the coil is an imprinted metallic shape on a fabric (e.g., silk) substrate or dressing.

It is contemplated that iEF cancer treatment will be free of harmful side effects such as those associated with chemotherapy, immunotherapy, and radiation.

In the present invention, a device for treatment of solid cancers, the device comprising an external portable induction coil. The device is characterized in that the device further comprises a power source operatively connected to the external portable induction coil and configured to generate an induced electric field between 1 mV/cm - 2.4 mV/cm peak field strength in the solid cancers by the external portable induction coil, and wherein the external portable induction coil is adapted to provide the induced electric field near or within a solid cancer.

This summary is provided to introduce a selection of concepts in a simplified form that are further described below in the detailed description. This summary is not intended to identify key features or essential features of the claimed subject matter, nor is it intended to be used to limit the scope of the claimed subject matter.

## DESCRIPTION OF THE DRAWINGS

The foregoing summary, as well as the following detailed description of illustrative embodiments, is better understood when read in conjunction with the appended drawings. For the purpose of illustrating the disclosed embodiments, there is shown in the drawings example constructions of the embodiments; however, the possible embodiments are not limited to the specific methods and instrumentalities disclosed. In the drawings:

FIG. 1A illustrates LDH activities in MDA-MB-231 cells with and without iEF and EGF;

FIG. 1B illustrates SDH activities in MDA-MB-231 cells with and without iEF and EGF;

FIG. 1C illustrates LDH activities in MCF10A cells with and without iEF and EGF;

FIG. 1D illustrates SDH activities in MCF10A cells with and without iEF and EGF;

FIGS. 2A-2C illustrate relative baseline oxygen consumption (OCR) of MDA-MB-231, MCF10CA1a, and MCF10A cells after 12 h of iEF treatment with or without 25 ng/mL EGF supplement;

FIGS. 2D-2F illustrate relative baseline glycolysis of MDA-MB-231, MCF10CA1a, and MCF10A cells after 12 h of iEF treatment with or without 25 ng/mL EGF supplement;

FIG. 3A illustrates a plastic mouse cage and a square cross-sectional coil that produces induced electric fields (iEF);

FIG. 3B illustrates Rms values of the iEF calculated using a COMSOL are shown here. The dotted region denotes the approximate location of the mice relative to the cage and the coil;

FIG. 4A illustrates a graph showing tumor volume measured every week after 2 weeks of cell injection showing no changes in primary tumor volumes;

FIG. 4B illustrates representative images of lungs harvested at the end of experimentation;

FIG. 4C illustrates a graph showing metastatic nodules on the lungs counted by dissection microscope;

FIGS. 5A-5C illustrate in vivo experimental results for the conditions shown in FIGS. 4A-4C;

FIGS. 6A-6C illustrate additional in vivo experimental results for the conditions shown in FIGS. 4A-4C;

FIG. 7 illustrates tumor lysates from control (Ctrl) and iEF treated mice analyzed for the presence of phosphorylated EGFR (Y1068) and expression of EMT markers Vimentin and E-cadherin, by western blots;

FIGS. 8A-8C illustrate examples of an iEF treatment device;

Fig. 9A illustrates representative photographs of the 4T1 primary mammary tumors;

FIG. 9B is a graph of the growth of the primary tumor as measured using a digital caliper;

FIG. 9C illustrates the weights of the primary tumors as measured at the end of iEF treatment;

FIG. 9D illustrates lung metastases as quantified by counting of foci per field of view;

FIGS 10A-10C illustrate single-cell suspension of tumor tissues;

FIGS. 10D-10E illustrate matched lungs analyzed for the enrichment of total macrophages and granulocytic myeloid-derived suppressor cells by using flow cytometry;

FIG. 11A illustrates analysis results for the infiltration of total CD8+ T cells (CD3+CD8+) after iEF treatment; and

FIG. 11B illustrates analysis results for the infiltration of activated (CD3+CD8+CD69+) CD8+ T cells after iEF treatment.

## DETAILED DESCRIPTION

The following description of the disclosure is provided as an enabling teaching of the disclosure in its best, currently known embodiment(s). To this end, those skilled in the relevant art will recognize and appreciate that many changes can be made to the various embodiments of the invention described herein, while still obtaining the beneficial results of the present disclosure. It will also be apparent that some of the desired benefits of the present disclosure can be obtained by selecting some of the features of the present disclosure without utilizing other features. Accordingly, those who work in the art will recognize that many modifications and adaptations to the present disclosure are possible and can even be desirable in certain circumstances and are a part of the present disclosure. Thus, the following description is provided as illustrative of the principles of the present disclosure and not in limitation thereof.

Unless defined otherwise, all technical and scientific terms used herein have the same meaning as commonly understood to one of ordinary skill in the art to which this invention belongs. As used in the specification and claims, the singular form "a," "an," and "the" include plural references unless the context clearly dictates otherwise. As used herein, the terms "can," "may," "optionally," "can optionally," and "may optionally" are used interchangeably and are meant to include cases in which the condition occurs as well as cases in which the condition does not occur. Reference in the specification to "one embodiment" or "an embodiment" or "an example embodiment" means that a particular feature, structure, or characteristic described is included in at least one embodiment described herein and does not imply that the feature, structure, or characteristic is present in all embodiments described herein.

Ranges can be expressed herein as from "about" one particular value, and/or to "about" another particular value. When such a range is expressed, another embodiment includes from the one particular value and/or to the other particular value. Similarly, when values are expressed as approximations, by use of the antecedent "about," it will be understood that the particular value forms another embodiment. It will be further understood that the endpoints of each of the ranges are significant both in relation to the other endpoint, and independently of the other endpoint. It is also understood that there are a number of values disclosed herein, and that each value is also herein disclosed as "about" that particular value in addition to the value itself. For example, if the value "10" is disclosed, then "about 10" is also disclosed.

Overview

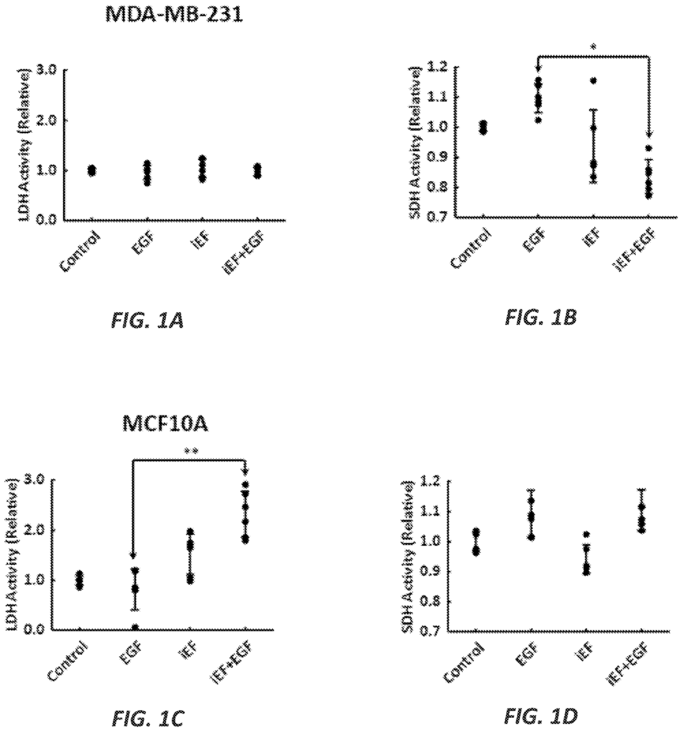

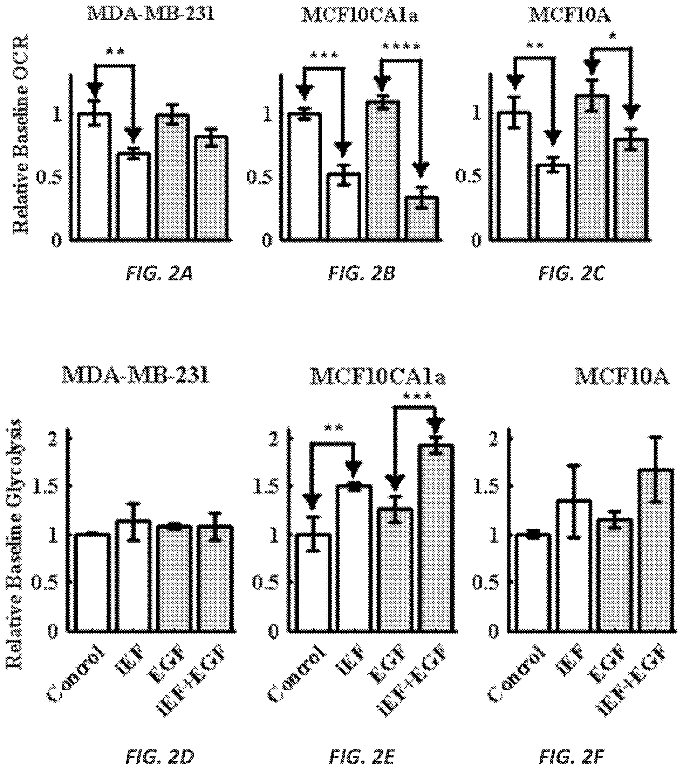

Oxidative phosphorylation in the mitochondria of metastatic breast cancer cells (MDA-MB-231) is suppressed by iEFs but not in the normal MCF10A breast cells. This is evidenced by FIG. 1A, which illustrates LDH activities in MDA-MB-231 cells with and without iEF and EGF. FIG. 1B illustrates SDH activities in MDA-MB-231 cells with and without iEF and EGF. FIG. 1C illustrates LDH activities in MCF10A cells with and without iEF and EGF. FIG. 1D illustrates SDH activities in MCF10A cells with and without iEF and EGF. In FIGS. 1A-1D, the values have been normalized to the control condition. Black circles represent the calculated activities from each well across 3 experiments. Error bars represent one standard deviation above and below the mean. *p < 0.05. In addition, FIGS. 2A-2C illustrate relative baseline oxygen consumption (OCR) of MDA-MB-231, MCF10CA1a, and MCF10A cells after 12 h of iEF treatment with or without 25 ng/mL EGF supplement. FIGS. 2D-2F illustrate relative baseline glycolysis of MDA-MB-231, MCF10CA1a, and MCF10A cells after 12 h of iEF treatment with or without 25 ng/mL EGF supplement. In FIGS. 2A-2F, the shaded bars represent conditions with EGF. * p < 0.05, ** p < 0.01, *** p < 0.001, **** p < 0.0001.

This demonstrates that iEFs may be used to hinder metastasis, which is an energy-intensive process. Though cancer cells are known to rely on other metabolic pathways such as glycolysis, fatty acid metabolism, and glutamine metabolism, they still rely primarily on oxidative phosphorylation to generate energy required for metastasis. Therefore, as disclosed herein, iEFs can be effective in attacking the most energetic pathways that cancer cells use to metastasize to distant sites.

Experimental Results in an Orthotopic mouse model

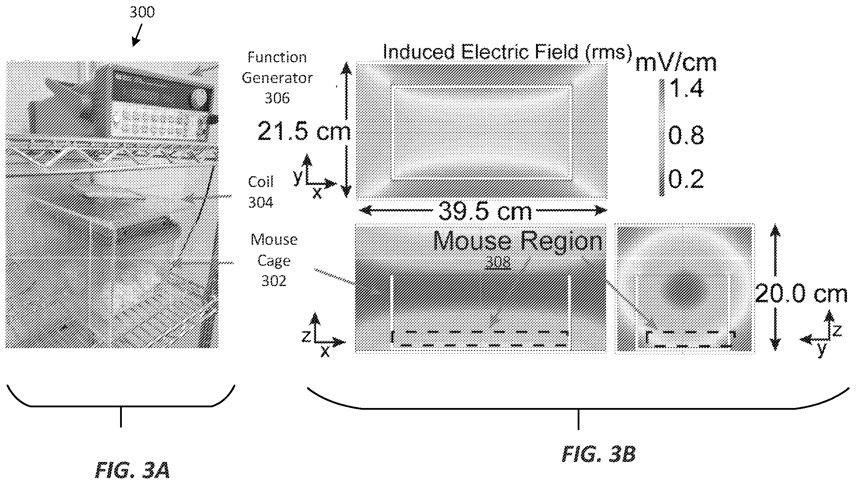

As mentioned above, induced electric fields play a role in selectively altering the metabolic pathways of breast cancer cells, hindering their capacity to migrate and replicate, thus rendering them less metastatic. In accordance with the present disclosure, this may be used to hinder tumor progression and metastasis. With reference to FIG. 3A, there is illustrated an example test environment 300 that includes mouse cage 302 and a square cross-sectional coil 304 that is used to produce electric fields (iEF) within the mouse cage 302. Also shown is a function generator 306 that was used to drive a 55 mA current through the Helmholtz- like coil. The environment 300 enables an experimental approach on the exterior of the mouse cage 302 in an *in vivo* mouse model of metastatic breast cancer.

The orthotopic mouse model recapitulates the various stages of metastatic breast cancer progression. In this model, highly metastatic mouse triple-negative breast cancer (TNBC) cells (Mvt1) are injected ($5x10^5$ cells in 100 μL PBS) orthotopically into the mammary fat pads of wild-type FVB background female mice (an albino inbred, laboratory, and immunocompetent (i.e., with intact immune system) mouse strain that is named because of its susceptibility to Friend leukemia Virus B) 6 weeks of age and metastasize into the lungs. The mice were placed in cages which themselves were placed within the housing 302 around which the Solenoid-like coil 304 is wrapped, as shown in FIG. 3A. In an implementation, the coil comprises 62 evenly spaced turns of 32 AWG insulated wire. The electrical properties of the coil were determined using an LCR meter to be L=437 μH and R=28 Ω. An electrical current of 55 mA (peak to peak) is driven through the coil using the function generator 306 that imposes a 100 kHz, 20 Vpp sawtooth waveform. The time-varying current produces a magnetic induction B, which was measured to be 11 μT at this current. As shown in FIG. 3B, the range of iEF at the base of the cage 302 where the mice are likely to be (shown as mouse region 308), is calculated to be 1 mV/cm - 2.4 mV/cm peak field and 0.7 to 1.2 mV/cm (or 700-1200 μV/cm) root mean square (rms) field strength. Treatment began 2 weeks after injection of cells and continued for five weeks. The diameter of the tumors was measured every week using an external digital caliper. Tumor volume was then calculated using the formula $V = LA \times SA^2 \times 0.52$ where LA is the largest and SA is the smallest superficial diameter.

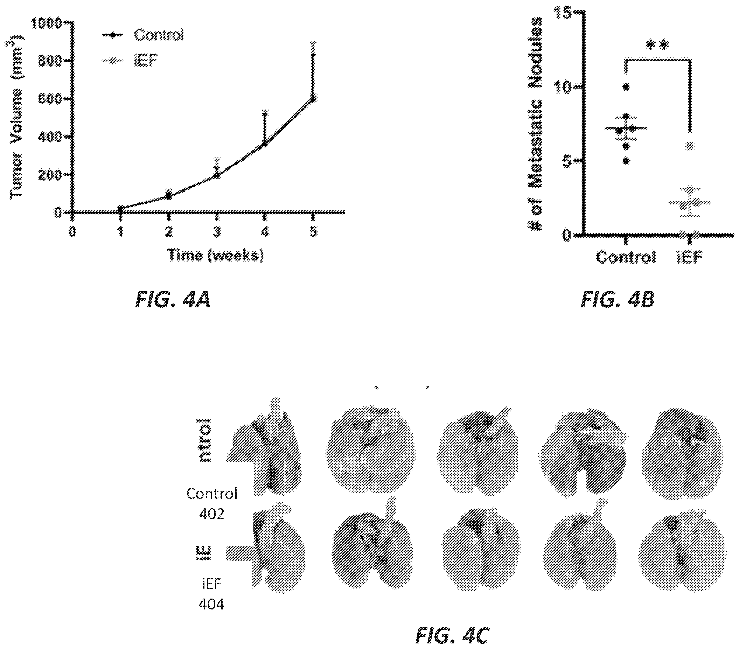

FIGS. 4-6 show results of applying iEF to the orthotopic mouse model in the apparatus shown in FIG. 3A. As discussed earlier, the TNBC cells injected directly into the mammary fat pads of these FVB mice eventually metastasize to the lungs. Unlike other models that involve subcutaneous or tail vein injection of cancer cells, in this orthotopic model, tumor progression occurs in the native breast microenvironment and metastasis from this primary site requires invasion, migration, and intravasation. As can be seen from Fig. 4A, the data shows that application of iEF does not affect the rate of growth of the primary mammary pad tumor *in vivo* over the period five weeks. However, there is a significant reduction in spontaneous metastasis of Mvt1 cells from mammary glands to the lungs as shown in FIG. 4B that illustrates a comparison of total metastatic nodules from each pair of lungs of treated and untreated mice. ** is p < 0.01 using Mann-Whitney U Test. In FIG. 4B, single data points represent total nodules from one pair of lungs. Error bars represent ±SEM. FIG. 4C shows images of lungs taken from untreated (Control 402) and treated (iEF 404) mice. These observations demonstrate a direct role of iEF treatment in inhibiting metastasis from the primary site by hindering cancer cell motility and subsequent dissemination to distant sites that is independent of primary tumor burden. Importantly, no adverse side effects in mice were observed due to iEF treatment.

Reduction in Tumor Associated Immune Cells

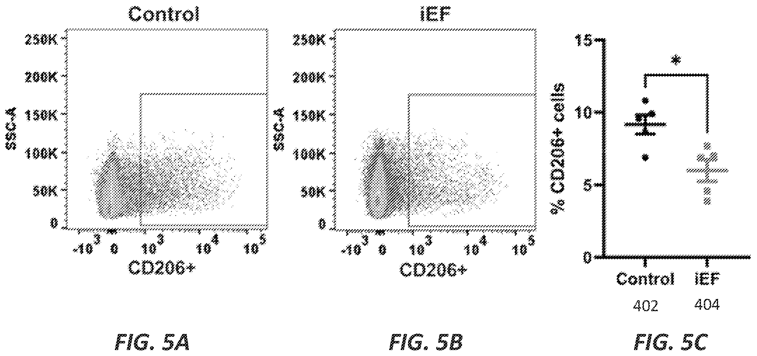

Tumor associated macrophages (TAMs) have been shown to be vital to the growth and dissemination of breast cancer primary tumors. Specifically, TAMs play a role in angiogenesis and intravasation of cancer cells leading to metastases. Cells from the primary tumors of the treated and untreated mice were analyzed for the presence of M2-type TAMs. Cells were tagged for CD45 and CD11b surface markers to identify CD45+/CD11b+ monocytes. FIG. 5A illustrates representative scatter plots of CD45+/CD11b+ gated monocytes plotted with side scatter on y-axis and CD206+ on x-axis. M2-TAMs were then identified based on the expression of the surface marker CD206. FIG. 5B illustrates the quantification of CD206+ subpopulation in both groups. It can be seen that there are significantly less M2-TAMs in iEF treated tumors. FIG. 5C illustrates a comparison of the untreated control 402 mice and treated iEF 404 mice where * is $p < 0.05$ using Mann-Whitney U Test. Data points shown in FIG. 5C represent values from each mouse and error bars represent $\pm$SEM.

Reduction in Cancer Stem Cells

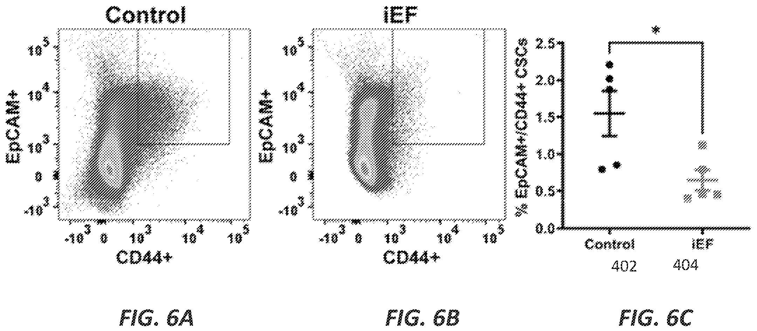

Breast cancers harbor a subset of cells with stem-like properties known as cancer stem-like cells or CSCs, with properties including self-renewal and drug resistance, that drive tumor initiation, recurrence, and metastasis. Breast CSCs are defined by markers aldehyde dehydrogenase (ALDH+) and CD24-/CD44+. These markers classify CSCs into more epithelial, proliferative cells (ALDH+); mesenchymal, invasive cells (CD24-/CD44+) that arise from epithelial-to-mesenchymal transition (EMT); and hybrid cells (ALDH+/CD24-CD44+) with plastic, intermediate phenotypes. The presence of cancer stem cells (CSCs) in the primary tumor has shown to have a strong correlation with metastases. While there is no causative link between CSCs and distant metastases, it is still an important prognostic tool. For example, FIG. 6A shows an identification of CD44+ and EpCAM+ CSCs extracted from the primary tumors. As shown in FIG. 6B, a percentage of CD44+/EpCAM+ cells is significantly lower in tumors from mice treated with iEF. FIG. 6C illustrates a comparison of the untreated control 402 mice and treated iEF 404 mice where * is $p < 0.05$ using Mann-Whitney U Test. Data points shown in FIG. 5C represent values from each mouse and error bars represent $\pm$SEM.

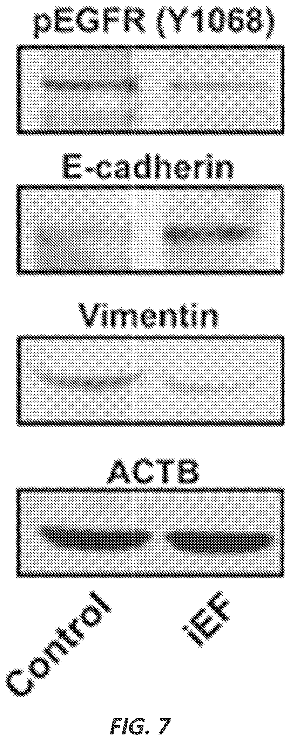

Lysates from the primary tumors were analyzed for expression of specific epithelial-mesenchymal transition (EMT) markers as well as phosphorylated EGFR. It can be seen from FIG. 7 that there is a lower expression of pEGFR. While the mechanism of this reduction is under investigation, it does correlate with a reduction on M2-TAMs present in the primary tumor. M2-TAMs are known to release EGF. Alternatively, iEFs have shown to reduce pEGFR in vitro on the TNBC cell line MDA-MB-231. In addition to pEGFR, expression of EMT markers E-cadherin and Vimentin were measured. The role of E-cadherin is complex as it is often assumed that the loss of E-cadherin results in the process of EMT. However, it has been shown that E-cadherin+/vimentin+ breast cancer patients have a worse overall survival when compared to E-cadherin-/vimentin-. This demonstrates the complexity of the disease. Importantly, E-cadherin+/vimentin- patients showed to have the greatest overall survival. It can be seen that iEFs increased E-cadherin expression and reduced vimentin expression simultaneously, which when taken together is a better overall prognosis. Thus, we have identified decreased populations of CSCs and decreased expression of EMT markers, complementing hindered metastases to the lungs in response to iEF treatment.

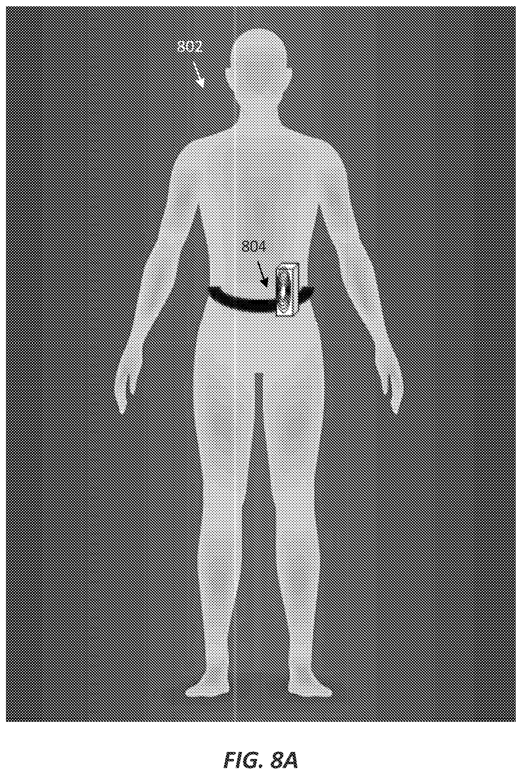

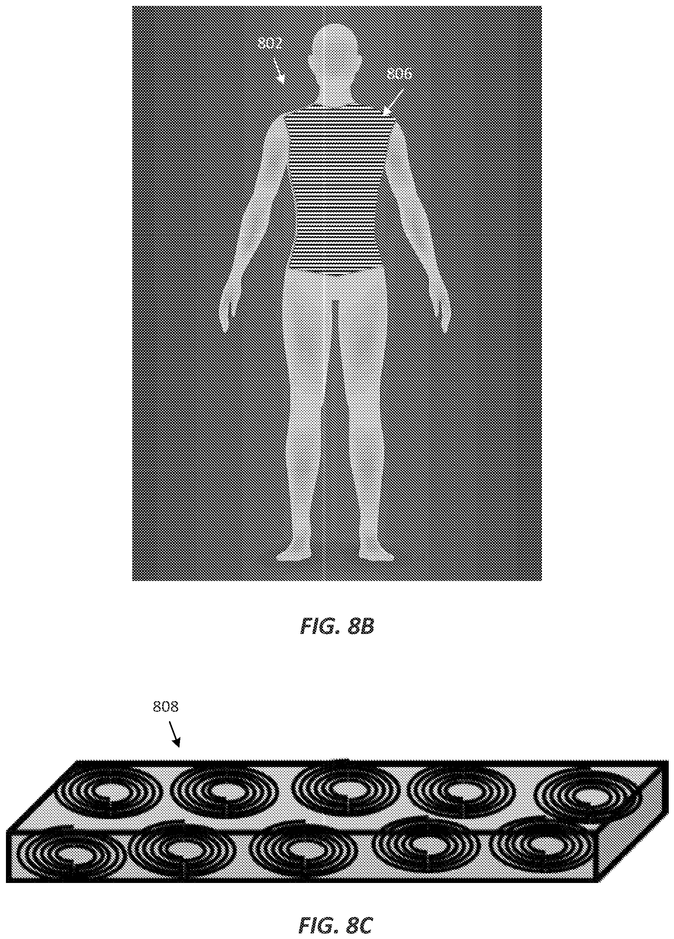

*In vitro* and *in vivo* results to date thus indicate that iEF treatment can be used to control and hinder metastatic disease without any accompanying adverse effects. The translation of iEF treatment for higher animals and humans can potentially be enabled in several forms. As shown in FIG. 8A, one example implementation that may be used for the treatment of a patient 802 and may involve an external, portable induction coil 804 with its associated power source. The patient 802 would continuously (or for a pre-determined duration) wear the external power supply and iEF coil 804 until treatment is completed as determined by standard diagnostic techniques (CT scans, PET scans, ultrasound, biopsy, etc.). As shown in FIG. 8B, another example implementation is the coil generating the iEF and its associated power supply being an integral part of a fabric 806 worn by the patient 802. FIG. 8C illustrates another example implementation where iEF generating coils are embedded in a mattress 808 on which the patient 802 would sleep or rest. It is anticipated that iEF cancer treatment will be free of the harmful side effects of chemotherapy, immunotherapy, and radiation.

*In vitro* and *in vivo* results to date indicate that iEF treatment can inhibit functions of chemokines and growth factors such CXCR4/CXCL12 and EGF/EGFR as well enchase the anti-tumor immune responses. The iEF treatment could be used in combination with other therapies including chemo, targeted and immunotherapy to enhance the therapeutic effects of these therapies.

Additional Experiment Results in an Orthotopic Mouse Model

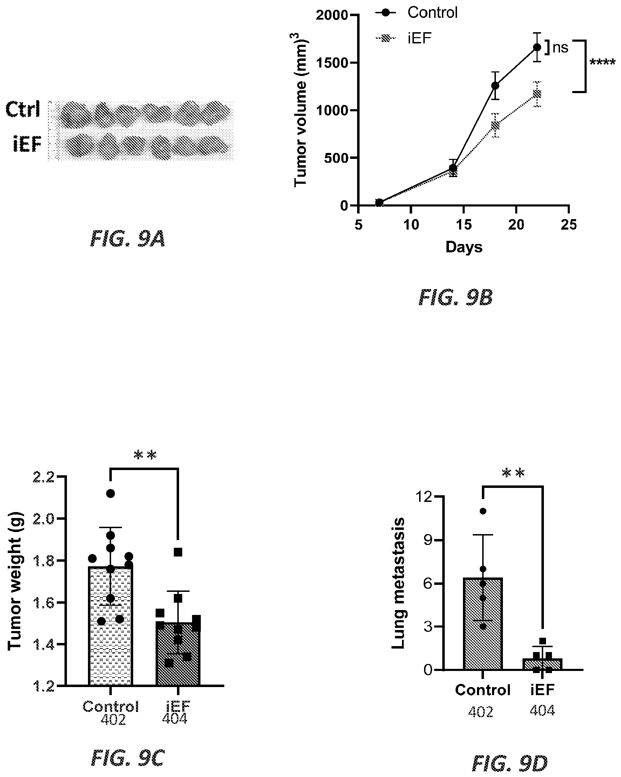

Additional experiments were conducted in an orthotopic mouse model (BALB/c wild-type immunocompetent mice) and applied iEF treatment (as described previously with the Mvt-1 mouse model) but with a more aggressive 4T1 cell line. The same iEF apparatus shown in FIG. 3A was used and the results described below are for 24h iEF continuous exposure post formation of palpable primary tumors, through the duration of the experiment. FIGS. 9-11 show the results of applying iEF to the orthotopic mouse model. As in the above experiment, TNBC cells are injected directly into the mammary fat pads of immunocompetent BALB/c mice that eventually metastasize to the lungs. As shown in FIGS. 9A-9D, the in-vivo data show that the application of iEF significantly reduced the rate of growth of the primary tumor. In addition, a significant reduction in spontaneous metastasis of 4T1 cells from mammary glands to the lungs was observed, as shown in FIG. 9D. In particular, FIG. 9A illustrates representative photographs of the 4T1 primary mammary tumors. FIG. 9B is a graph showing the growth of the primary tumor as measured using a digital caliper. FIG. 9C shows the weights of the primary tumors as measured at the end of iEF treatment. FIG. 9D illustrates lung metastases as quantified by counting of foci per field of view. In FIGS. 9C and 9D, the Ctrl 402 were untreated mice and the iEF were mice treated by the induced electric field. * P<0.05; ** P < 0.01; **** P<0.0001

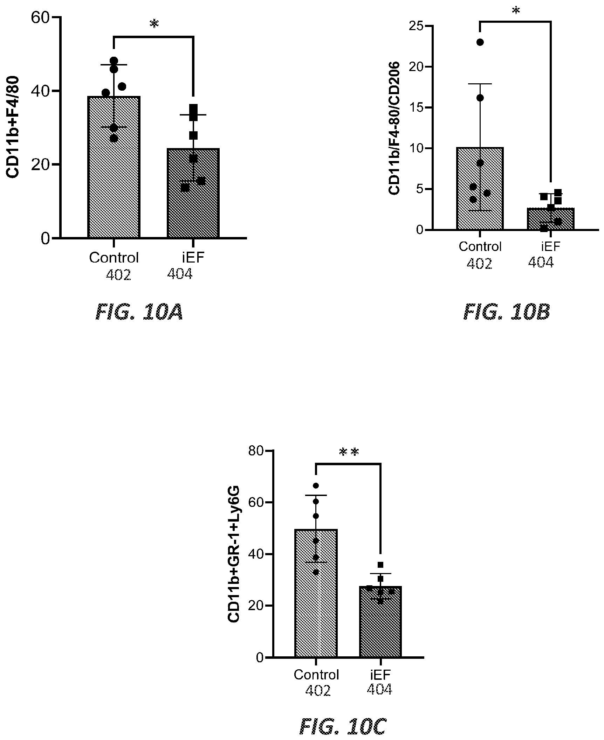

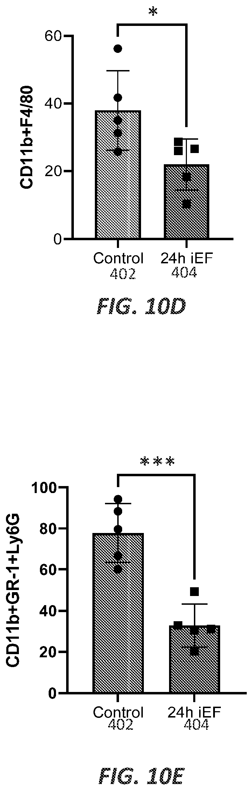

As shown in FIGS. 10A-10E, iEF treatment also reduced the recruitment of tumor-supporting immune cells including macrophages and granulocytic (Ly6G+) MDSCs both in the tumor and its metastatic site lung. As shown in FIG. 10A, iEF application reduced the recruitment of pro-tumor M2 TAMs in the breast TME. In particular, FIGS. 10A-10E show analysis of single-cell suspension of tumor tissues and matched lungs for the enrichment of total macrophages (CD11b+F4/80+), M2 TAMs (CD11b+F4/80+CD206+), and granulocytic myeloid-derived suppressor cells (MDSCs) (CD11b+GR-1+Ly6G+) by using flow cytometry.

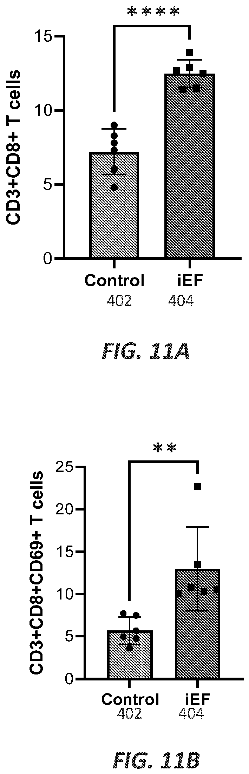

As shown FIGS. 11A-11B, iEF treatment significantly increased the tumor infiltration of activated (CD69+) CD8+ T cells, which plays a role in tumor clearance. CD69 is a classical marker of lymphocyte activation and is required for the recruitment and maintenance of T cells. These observations suggest a direct role of iEF treatment in inhibiting tumor growth and metastasis by altering the tumor microenvironment and migratory potential of cancer cells.

Thus, the iEF treatment reduced breast tumor progression and metastasis. iEFs application also significantly reduced the infiltration of macrophages and MDSCs both in the primary tumor and its secondary site. Moreover, iEFs significantly reduced the infiltration of M2 TAMs in the TME. Furthermore, the present disclosure shows that iEFs augmented the recruitment of activated CD8+ T cells in the TME. Myeloid-derived suppressor cells (MDSCs) are a heterogeneous population of myeloid cells that support tumor growth and metastasis. MDSCs are known to promote tumor-associated immunosuppressive effects in the TME. MDSCs play a crucial role in diminishing the efficacy of immunotherapies and enhancing drug resistance. Therefore, iEF treatment could be used to inhibit the recruitment and polarization of immune-suppressive myeloid cells while increasing the infiltration of CD8+ T cells in the breast TME, to improve the overall anti-tumor immunity thereby reducing breast tumor growth and metastasis.

Conclusion

The treatment with iEF disclosed herein did not show any negative effects on mice. In-vitro and in-vivo results strongly suggest that inhibiting the growth and metastasis of breast cancer using iEF treatment is highly safe and effective.

Numerous characteristics and advantages provided by aspects of the present invention have been set forth in the foregoing description together with details of structure and function.

The scope of the present invention is determined by the appended claims.

**Claims**

1.    A device for treatment of solid cancers, the device comprising:

an external portable induction coil (304, 804); and
a power source operatively connected to the external portable induction coil (304, 804) and configured to generate an induced electric field between 1 mV/cm - 2.4 mV/cm peak field strength in the solid cancers by the external portable induction coil (304, 804),
wherein the external portable induction coil (304, 804) is adapted to provide the induced electric field within the solid cancers.

2.    The device of claim 1, wherein the external portable induction coil (304, 804) and the associated power source is configured to be worn by a patient (802) having the solid cancer.

3.    The device of claim 2, wherein the external portable induction coil (304, 804) and the associated power source is configured to be worn by the patient (802) continuously until treatment of the solid cancer is completed.

4.    The device of claim 2, wherein the external portable induction coil (304, 804) and the associated power source is configured to be worn by the patient (802) for a predetermined duration.

5. The device of claim 1, wherein the external portable induction coil (304, 804) and the associated power source is integral to a fabric (806) worn by a patient (802) having the solid cancer.

6. The device of claim 1, wherein the external portable induction coil (304, 804) and the associated power source are embedded in a mattress (808) or other piece of furniture or object, on which a patient (802) having the solid cancer would sleep or rest.

7. The device of claim 1, wherein an electric current within the external portable induction coil (304, 804) is approximately 55 mA.

8. The device of claim 1, wherein a 100 kHz, 20 Vpp sawtooth waveform is used to generate the induced electric field.

9. The device of claim 1, wherein the external portable induction coil (304, 804) has an inductance of approximately 437 $\mu$H and a resistance of approximately 28 $\Omega$.


**Patentansprüche**

1. Vorrichtung zur Behandlung von soliden Tumoren, die Vorrichtung umfassend:

   eine externe tragbare Induktionsspule (304, 804); und
   eine Energiequelle, die funktionsfähig mit der externen tragbaren Induktionsspule (304, 804) verbunden und konfiguriert ist, um ein induziertes elektrisches Feld mit einer Spitzenfeldstärke zwischen 1 mV/cm und 2,4 mV/cm in den soliden Tumoren durch die externe tragbare Induktionsspule (304, 804) zu erzeugen, wobei die externe tragbare Induktionsspule (304, 804) geeignet ist, das induzierte elektrische Feld innerhalb der soliden Tumoren bereitzustellen.

2. Vorrichtung nach Anspruch 1, wobei die externe tragbare Induktionsspule (304, 804) und die zugehörige Energiequelle konfiguriert sind, um von einem Patienten (802) mit dem soliden Tumor getragen zu werden.

3. Vorrichtung nach Anspruch 2, wobei die externe tragbare Induktionsspule (304, 804) und die zugehörige Energiequelle konfiguriert sind, um vom Patienten (802) kontinuierlich getragen zu werden, bis die Behandlung des soliden Tumors abgeschlossen ist.

4. Vorrichtung nach Anspruch 2, wobei die externe tragbare Induktionsspule (304, 804) und die zugehörige Energiequelle konfiguriert sind, um vom Patienten (802) für eine vorbestimmte Dauer getragen zu werden.

5. Vorrichtung nach Anspruch 1, wobei die externe tragbare Induktionsspule (304, 804) und die zugehörige Energiequelle in ein Gewebe (806) integriert sind, das von einem Patienten (802) mit dem soliden Tumor getragen wird.

6. Vorrichtung nach Anspruch 1, wobei die externe tragbare Induktionsspule (304, 804) und die zugehörige Energiequelle in eine Matratze (808) oder ein anderes Möbelstück oder einen Gegenstand eingebettet sind, auf dem ein Patient (802) mit dem soliden Tumor schlafen oder ruhen würde.

7. Vorrichtung nach Anspruch 1, wobei ein elektrischer Strom innerhalb der externen tragbaren Induktionsspule (304, 804) etwa 55 mA beträgt.

8. Vorrichtung nach Anspruch 1, wobei eine 100 kHz, 20 Vpp Sägezahnwellenform verwendet wird, um das induzierte elektrische Feld zu erzeugen.

9. Vorrichtung nach Anspruch 1, wobei die externe tragbare Induktionsspule (304, 804) eine Induktivität von etwa 437 $\mu$H und einen Widerstand von etwa 28 $\Omega$ aufweist.


**Revendications**

1. Dispositif de traitement de cancers solides, le dispositif comprenant :

une bobine d'induction portable externe (304, 804) ; et

une source d'alimentation reliée de manière fonctionnelle à la bobine d'induction portable externe (304, 804) et configurée pour générer un champ électrique induit d'une intensité de champ maximale comprise entre 1 mV/cm et 2,4 mV/cm dans les cancers solides par la bobine d'induction portable externe (304, 804),

dans lequel la bobine d'induction portable externe (304, 804) est adaptée pour fournir le champ électrique induit à l'intérieur des cancers solides.

2. Dispositif selon la revendication 1, dans lequel la bobine d'induction portable externe (304, 804) et la source d'alimentation associée sont configurées pour être portées par un patient (802) atteint d'un cancer solide.

3. Dispositif selon la revendication 2, dans lequel la bobine d'induction portable externe (304, 804) et la source d'alimentation associée sont configurées pour être portées par le patient (802) en continu jusqu'à la fin du traitement du cancer solide.

4. Dispositif selon la revendication 2, dans lequel la bobine d'induction portable externe (304, 804) et la source d'alimentation associée sont configurées pour être portées par le patient (802) pendant une durée prédéterminée.

5. Dispositif selon la revendication 1, dans lequel la bobine d'induction portable externe (304, 804) et la source d'alimentation associée sont intégrées à un tissu (806) porté par un patient (802) atteint d'un cancer solide.

6. Dispositif selon la revendication 1, dans lequel la bobine d'induction portable externe (304, 804) et la source d'alimentation associée sont intégrées dans un matelas (808) ou un autre meuble ou objet, sur lequel un patient (802) atteint d'un cancer solide dormirait ou se reposerait.

7. Dispositif selon la revendication 1, dans lequel un courant électrique à l'intérieur de la bobine d'induction portable externe (304, 804) est d'environ 55 mA.

8. Dispositif selon la revendication 1, dans lequel une forme d'onde en dents de scie de 100 kHz et 20 Vpp est utilisée pour générer le champ électrique induit.

9. Dispositif selon la revendication 1, dans lequel la bobine d'induction portable externe (304, 804) présente une inductance d'environ 437 $\mu$H et une résistance d'environ 28 $\Omega$.

## MDA-MB-231

**FIG. 1A**

**FIG. 1B**

## MCF10A

**FIG. 1C**

**FIG. 1D**

FIG. 2A

FIG. 2B

FIG. 2C

FIG. 2D

FIG. 2E

FIG. 2F

Induced Electric Field (rms) mV/cm

1.4
0.8
0.2

39.5 cm

Mouse Region
308

20.0 cm

z
y

r

z
x

Function Generator 306

21.5 cm

y
x

Coil 304

Mouse Cage 302

300

*FIG. 3A*

*FIG. 3B*

*FIG. 4A*

*FIG. 4B*

*FIG. 4C*

FIG. 5A

FIG. 5B

FIG. 5C

Control

EpCAM+

CD44+

FIG. 6A

iEF

EpCAM+

CD44+

FIG. 6B

% EpCAM+/CD44+ CSCs

*

Control
402

iEF
404

FIG. 6C

FIG. 7

FIG. 8A

FIG. 8B

FIG. 8C

FIG. 9A

FIG. 9B

FIG. 9C

FIG. 9D

FIG. 10A

FIG. 10B

FIG. 10C

*FIG. 10D*

*FIG. 10E*

*FIG. 11A*

*FIG. 11B*

**REFERENCES CITED IN THE DESCRIPTION**

**Patent documents cited in the description**

- WO 2021149864 A
- US 2019299019 A